# EUROPÄISCHE PATENTANMELDUNG

(11) **EP 0 852 937 A2**
(43) Veröffentlichungstag der Anmeldung: **15.07.1998**
(21) Anmeldenummer: 97109928.8
(22) Anmeldetag: 18.06.1997
(51) Int. Cl.: A61F 9/04

(54) **Schutzbrille**

(30) Priorität: 10.01.1997 DE 29700231 U
(71) Anmelder: Hofmann, Sybille, 35463 Fernwald-Steinbach (DE)
(72) Erfinder: Hofmann, Hans-Jörg, 35463 Fernwald-Steinbach (DE)
(74) Vertreter: Missling, Arne, Dipl.-Ing.

(57) **Zusammenfassung**

Die insbesondere in Solarien bei der künstlichen Hautbräunung verwendeten Schutzbrillen verdecken auch mit ihrem Nasenbügel einen Teil der Nasenwurzel, so daß dort eine ungebräunte, Partie verbleibt, die ohne Brille ins Auge fällt. Die Erfindung verwendet statt dessen einen Nasenbügel aus transparentem Kunststoff, der zusammen mit einer Berandung der Schalen für die Schutzgläser oder spezieller Deckschalen das Brillengestell bildet und für eine ungestörte Bräunung der Haut auch im Bereich der Nase sorgt.

## Beschreibung

Die Erfindung betrifft eine Schutzbrille mit von einem Brillenrahmen gehaltenen Schutzgläsern, insbesondere zum Schutz der Augen während der Einwirkung eines Lichtstromes auf einer Bräunungsbank zur Erzeugung einer künstlichen Sonnenbräune der Haut, wobei die Schutzgläser jeweils von einer lichtundurchlässigen Schale gefaßt sind, deren sphärische, der Form des Augapfels ungefähr folgende Kontur den Einfall von Licht in das Auge verhindert, wobei eine Berandung an der Augenhöhle anliegt oder deren Form mit geringem Abstand folgt und die Berandungen durch einen Nasenbügel miteinander verbunden sind.

In der Regel ist eine derartige Schutzbrille als Klemmer ausgebildet, bei der der Nasenbügel am Nasenrücken eines Probanden klemmbar ist, so daß, beispielsweise bei der Benutzung einer Bräunungsbank, die übrige Gesichtspartie dem auftreffenden Lichtstrom ausgesetzt ist und eine homogene Gesichtsbräune entsteht. Die Schutzgläser verhindern dabei eine Schädigung der Augen, und die entsprechend ausgeformten Schalen sorgen dafür, daß selbst bei Schutzgläsern relativ kleinen Durchmessers kein Licht in die Augen einstrahlen kann.

Die Schalen und der Nasenbügel bestehen, den Brillenrahmen ausbildend, einstückig aus einem lichtundurchlässigen Kunststoff und sind auf diese Weise, beispielsweise mittels eines Spritzwerkzeuges, bequem herstellbar; die Schutzgläser werden danach in geeigneter Weise in den Schalen befestigt. An den Schalen können auf ihrer jeweils dem Nasenbügel abgewandten Ende Ösen vorgesehen sein, so daß die Schutzbrille, beispielsweise mittels Haltebügeln, Haltefäden oder dergleichen, an den Ohrmuscheln eines Probanden gehaltert werden kann.

Der Nasenbügel unterbindet allerdings die freie Immission eines auftreffenden Lichtstromes in dem von dem Nasenbügel verdeckten Bereich der Nasenwurzel, so daß dort eine ausreichende, mit den übrigen Gesichtspartien korrespondierende Bräunung der Haut verhindert wird. Obwohl auch die Augenhöhlen durch die Schalen von einer vollständigen Bräunung ausgenommen sind, so ist doch der von dem Nasenbügel verursachte ungebräunte Bereich der Nasenwurzel viel unangenehmer, weil er auf der ungestörten Hautfläche der Nase viel leichter ins Auge fällt als die Hautbereiche der Augenhöhlen.

Die Erfindung hat sich deshalb die Aufgabe gestellt, diese Mängel abzustellen und eine Schutzbrille der eingangs genannten Art so auszubilden, daß ein Lichtstrom auch im Bereich der Nase ungestört auf die Gesichtshaut eines Probanden einwirken kann.

Erfindungsgemäß wird die Aufgabe dadurch gelöst, daS der Nasenbügel aus einem für ultraviolettes Licht durchlässigen Werkstoff besteht, ebenso wie in weiterer Ausbildung der Erfindung auch die Berandung.

Eine solche, die geschilderten Mängel in einfacher Weise beseitigende Anordnung läßt sich leicht ausführen, wenn die Berandung an den Schalen ausgebildet ist, wobei es günstig ist es, wenn die Schalen einschließlich der Berandung und der Nasenbügel einstückig den Brillenrahmen ausbilden. Eine solche Verbundkonstruktion kann mit einfachen Mitteln hergestellt werden und umfaßt gegebenenfalls auch noch Ösen für Haltebügel oder dergleichen zur Ohrbefestigung der Schutzbrille an der Berandung, so daß die Schalen selbst sehr einfach gestaltet werden könne.

Es ist statt dessen aber auch möglich, daß die Berandungen einstückig an jeweils einer die zugehörige Schale lose überfangenden Deckschale ausgebildet sind; die Deckschalen folgen vorteilhaft jeweils der sphärische Kontur der an ihnen befestigten schalen, dieser Kontur benachbart oder an ihr anliegend. Ganz besonders bevorzugt ist dabei eine Ausführung, bei der der Nasenbügel und die Deckschalen einstückig den Brillenrahmen ausbilden. Insgesamt hat eine solche erfindungsgemäße Ausbildung einer Schutzbrille den Vorteil, daß eine Verbundkonstruktion nicht erforderlich ist: die lichtundurchlässigen Schalen werden gesondert hergestellt, der einheitlich aus einem lichtdurchlässigen Werkstoff bestehende Rahmen ist damit viel leichter herstellbar. Es ist zweckmäßig, wenn die Deckschalen und/oder die Schalen jeweils eine konstante Schalendicke aufweisen.

Eine erfindungsgemäße Schutzbrille ist besonders leicht herstellbar und praktisch zu handhaben, wenn für den Nasenbügel und/oder die Berandungen und/oder die Deckschalen ein transparenter Kunststoff verwendet wird.

Am besten ist es, wenn die Schutzbrille als Klemmer ausgebildet und der Nasenbügel am Nasenrücken eines Probanden klemmbar ist.

Die Erfindung wird nachstehend an Hand der Zeichnung an einem Ausführungsbeispiel näher erläutert. Es zeigen
- Fig.1: eine erfindungsgemäße Schutzbrille in der Gebrauchslage senkrecht zum einfallenden Lichtstrom in einer ersten Ausführung,
- Fig.2: eine Druntersicht zu Fig.1,
- Fig.3: eine erfindungsgemäße Schutzbrille in der Gebrauchslage senkrecht zum einfallenden Lichtstrom in einer anderen Ausführung und
- Fig.4: eine Druntersicht zu Fig.3.

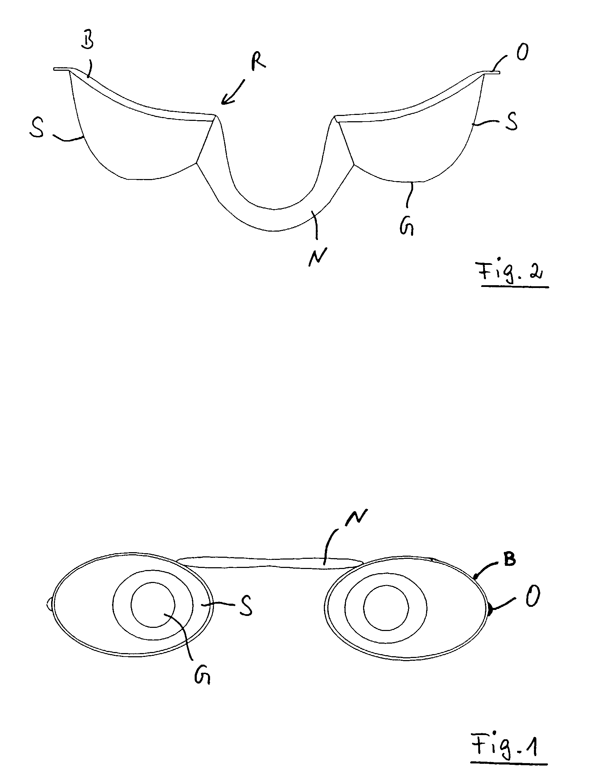

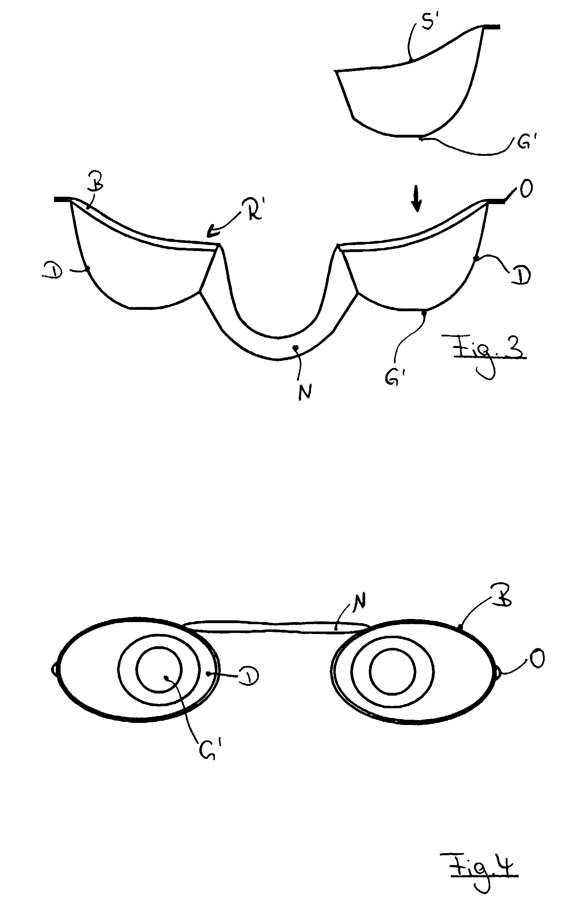

Eine Schutzbrille gemäß der Erfindung besteht in einer ersten Ausführung gemäß Fig.1,2 aus einem Brillenrahmen R, der einstückig aus einem Nasenbügel N und beiderseits je einer Berandung B einer lichtundurchlässigen Schale S gebildet ist und aus einem transparenten und zumindest für ultraviolettes Licht durchlässigen Werkstoff hergestellt ist. In die Schalen S sind Schutzgläser G eingesetzt, welche die Augen eines Probanden schützen, wenn dieser einem Lichtstrom ausgesetzt ist, beispielsweise auf einer Bräunungsbank in einem Solarium zu Erzeugung einer künstlichen Sonnenbräune der Haut. Die Berandungen B liegen möglichst leicht an den Augenhöhlen des Probanden an oder folgen deren Kontur mit geringem Abstand.

Eine weitere Ausbildung der Erfindung entsprechend Fig.3,4 verwendet statt dessen (wiederum lichtundurchlässige) Schalen S', die separat von dem Brillenrahmen R' angefertigt sind und von denen ein Exemplar vor dem Zusammenbau mit der zugehörigen Deckschale D in der Fig.1 dargestellt ist, in die sie in Pfeilrichtung eingesetzt wird; die Konturen der Schalen S' und der Deckschalen D stimmen soweit überein, daß sie ohne Schwierigkeiten ineinander gesteckt und miteinander in geeigneter Weise verbunden werden können; zumindest die Deckschalen D sind deshalb mit relativ geringer, konstanter Schalendicke ausgeführt; im Bereich der Schutzgläser G sind die Deckscheiben D jeweils mit einer Ausnehmung G' versehen, so daß ein ungestörter Durchblick durch die Schutzgläser G möglich ist.

Die Fig.4 zeigt den gesamten Brillenrahmen R', der einstückig und homogen mit den Berandungen B, den an den Berandungen B anschließenden Deckschalen D und dem Nasenbügel N aus dem gleichen, für ultraviolettes Licht durchlässigen Werkstoff besteht, im Gegensatz zu den lichtundurchlässigen Schalen S'.

Die Schutzbrille ist durchweg als Klemmer ausgeführt, bei dem der Nasenbügel N an der Nasenwurzel des Probanden festklemmbar ist. Trotzdem ist auch eine Ohrmuschel-Befestigung möglich: hierzu sind an den Berandungen B jeweils Ösen O vorgesehen, in denen Haltebügel, Haltefäden oder dergleichen einsetzbar sind.

### Bezugszeichen

- B: Berandung
- G: Schutzglas
- G': Ausnehmung
- N: Nasenbügel
- O: Öse
- R,R': Brillenrahmen

## Patentansprüche

1. Schutzbrille mit von einem Brillenrahmen (R,R') gehaltenen Schutzgläsern (G), insbesondere zum Schutz der Augen während der Einwirkung eines Lichtstromes auf einer Bräunungsbank zur Erzeugung einer künstlichen Sonnenbräune der Haut, wobei die Schutzgläser (G) jeweils von einer lichtundurchlässigen Schale (S,S') gefaßt sind, deren sphärische, der Form des Augapfels ungefähr folgende Kontur den Einfall von Licht in das Auge verhindert, wobei eine Berandung (B) an der Augenhöhle anliegt oder deren Form mit geringem Abstand folgt und die Berandungen (B) durch einen Nasenbügel (N) miteinander verbunden sind, dadurch gekennzeichnet, daß der Nasenbügel (N) aus einem für ultraviolettes Licht durchlässigen Werkstoff besteht.

2. Schutzbrille nach Anspruch 1, dadurch gekennzeichnet, daß die Berandung (B) aus einem für ultraviolettes Licht durchlässigen Werkstoff besteht.

3. Schutzbrille nach Anspruch 1 oder 2, dadurch gekennzeichnet, daß die Berandung (B) an den Schalen (S) ausgebildet ist.

4. Schutzbrille nach Anspruch 3, dadurch gekennzeichnet, daß die Schalen (S) einschließlich der Berandung (B) und der Nasenbügel (N) einstückig den Brillenrahmen (R) ausbilden.

5. Schutzbrille nach Anspruch 1 oder 2, dadurch gekennzeichnet, daß die Berandungen (B) einstückig an jeweils einer die zugehörige Schale (S') überfangenden Deckschale (D) ausgebildet sind.

6. Schutzbrille nach Anspruch 5, dadurch gekennzeichnet, daß die Deckschalen (D) jeweils der sphärische Kontur der an ihnen befestigten Schalen (S') folgen, dieser Kontur benachbart oder an ihr anliegend.

7. Schutzbrille nach einem der Ansprüche 5 oder 6, dadurch gekennzeichnet, daß der Nasenbügel (N) und die Deckschalen (D) einstückig den Brillenrahmen (R') ausbilden.

8. Schutzbrille nach einem der Ansprüche 5 bis 7, dadurch gekennzeichnet, daß die Deckschalen (D) eine konstante Schalendicke aufweisen.

9. Schutzbrille nach einem der Ansprüche 1 bis 8, dadurch gekennzeichnet, daß die Schalen (S,S') eine konstante Schalendicke aufweisen.

10. Schutzbrille nach einem der Ansprüche 1 bis 9, dadurch gekennzeichnet, daß für den Nasenbügel (N) und/oder die Berandungen (B) und/oder die Deckschalen (D) ein transparenter Kunststoff verwendet wird.

11. Schutzbrille nach einem der Ansprüche 1 bis 10, dadurch gekennzeichnet, daß sie als Klemmer ausgebildet und der Nasenbügel (N) an dem Nasenrücken eines Probanden klemmbar ist.
